# EUROPEAN PATENT APPLICATION

(11) **EP 4 843 569 A1**
(43) Date of publication of application: **25.11.2026**
(21) Application number: 26179440.8
(22) Date of filing: 19.05.2026
(51) Int. Cl.: A61K 31/198, A61K 33/06, A61P 7/04

(54) **A HEMOSTATIC COMPOSITION COMPRISING AMINO ACID-METAL COMPLEXES AND ITS USE THEREOF**

(30) Priority: 20.05.2025 US 202563808672 P
(71) Applicant: Amelio Biomedical Co., Ltd., New Taipei City 221425 (TW)
(72) Inventor: LI, I-CHEN, 221425 New Taipei City (TW); CHEN, CHI-JUNG, 221425 New Taipei City (TW)
(74) Representative: Rapisardi, Mariacristina

(57) **Abstract**

The present disclosure provides a hemostatic composition comprising an amino acid-metal complex, wherein the amino acid is glutamic acid or aspartic acid, and the metal is a divalent metal ion; the present disclosure also provides a use of the hemostatic composition for promoting hemostasis. The hemostatic composition of the present disclosure primarily promotes hemostasis through physical action, including adsorption of blood and formation of a stable structure at the wound, without primarily relying on coagulation factor activation mechanisms. Compared to traditional drugs or medical devices used for hemostasis, it can effectively reduce clotting time and blood loss, and has better biocompatibility, reducing the risk of allergic reactions and tissue irritation, and improving hemostatic efficiency and safety.

## Description

### BACKGROUND OF THE INVENTION

### 1. Field of the Invention

The present disclosure relates to a hemostatic composition, and in particular to a hemostatic composition that improves bleeding symptoms through the use of amino acid-metal complexes.

### 2. Description of the Related Art

Most clinically available hemostatic medical devices utilize chemical or physical mechanisms to promote hemostasis. For example, QuickClot primarily uses kaolin (a negatively charged silicate) to adsorb and activate coagulation Factor XII in the organism, thereby stimulating the coagulation pathway in the body. This ultimately leads to the production of large amounts of thrombin and the formation of a fibrin clot, which helps achieve hemostasis at the wound.

Another common hemostatic medical device is CeloX, which works by incorporating chitosan (a cationic polysaccharide) into its formulation. The chitosan binds to negatively charged red blood cells, platelets, or plasma proteins in the blood to form a gel-like substance, thereby sealing the wound and achieving hemostasis. In addition, some technologies utilize tranexamic acid, which inhibits fibrinolysis to prevent the breakdown of blood clots, thereby increasing the stability of the coagulation.

### BRIEF SUMMARY OF THE INVENTION

However, the aforementioned prior arts each have their own limitations and drawbacks. For example, kaolin relies on the activation of the individual's own coagulation factors; therefore, it cannot produce significant effects in individuals experiencing massive blood loss, taking anticoagulant medications, or with coagulopathy. Chitosan may trigger allergic reactions in individuals with shellfish allergies and leaves residues in wounds, often requiring debridement for removal, thereby increasing the risk of infection. Meanwhile, the use of tranexamic acid may lead to side effects such as thrombosis, and it lacks the ability to physically seal the wound; furthermore, as it is a pharmaceutical ingredient, its efficacy is also limited by tissue conditions or dilution by blood flow.

Therefore, given the shortcomings of existing hemostatic medical devicessuch as their reliance on the host's coagulation system, the risk of allergic reactions, lack of sealing capability, difficulty in removal, and potential for infection-it is particularly important to develop a hemostatic composition that combines effective hemostasis with high biocompatibility to meet clinical hemostatic needs.

Based on the aforementioned limitations and defects of the prior art, the present disclosure provides a hemostatic composition comprising an amino acid-metal complex; where the amino acid is glutamic acid or aspartic acid, and the metal is a divalent metal ion.

According to the embodiments of the present disclosure, the divalent metal ion is magnesium, manganese, cobalt, zinc, or calcium.

According to the embodiments of the present disclosure, the amino acid-metal complex is magnesium glutamate or magnesium aspartate, the structures of which are respectively represented by the following formula (I) and formula (II) below:

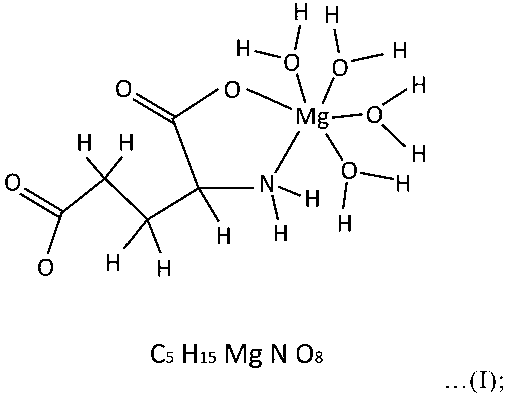

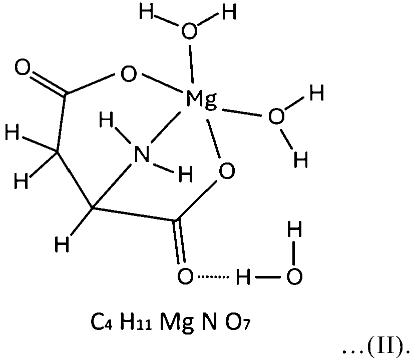

According to the embodiments of the present disclosure, the hemostatic composition does not contain chloride ions, sulfate ions, irritating salts, or antibiotics.

According to the embodiments of the present disclosure, the hemostatic composition is a powder, tablet, dressing, spray, or gel.

According to the embodiments of the present disclosure, the amino acid-metal complex is a freeze-dried powder.

According to the embodiments of the present disclosure, a pH value of the amino acid-metal complex after redissolving in water is 6.5 to 8.5.

According to the embodiments of the present disclosure, the amino acid-metal complex is a freeze-dried magnesium aspartate powder, and exhibits an amorphous form, a partially crystalline form, or a mixture thereof in X-ray diffraction analysis.

According to the embodiments of the present disclosure, the amino acid-metal complex is a freeze-dried magnesium aspartate powder, and comprises a peak at a 2θ value of 6.9° ± 0.2° in an X-ray powder diffraction pattern.

According to the embodiments of the present disclosure, the amino acid-metal complex contains a crystalline form of magnesium aspartate, which is formed by a magnesium aspartate complex under an alkaline condition.

The use of the hemostatic composition as described above is for improving bleeding symptoms.

In summary, the present disclosure provides a hemostatic composition comprising an amino acid-metal complex, which is used to improve bleeding symptoms. Compared to the medical devices or drugs used for hemostasis in the prior art, the amino acid-metal complex used in the present disclosure promotes hemostasis through physical action, has high biocompatibility, and reduces the risk of allergic reactions and tissue irritation. Furthermore, the hemostatic composition of the present disclosure also has significant improvements in clotting time and blood loss control.

### BRIEF DESCRIPTION OF THE DRAWINGS

FIG. 1 shows an X-ray powder diffraction pattern of the freeze-dried magnesium aspartate powder of the present disclosure.
FIG. 2 shows a single-crystal X-ray diffraction pattern of the magnesium aspartate crystal of the present disclosure.
FIG. 3 shows the hemostatic effect of different amino acid-metal complexes of the present disclosure in a mouse tail truncation model, including clotting time and blood loss, along with a comparison of the results with the control group and reference group.
FIG. 4 shows the hemostatic effect of different amino acid-metal complexes of the present disclosure in a rat tail truncation model, including clotting time and blood loss, along with a comparison of the results with the control group and reference group.

### DETAILED DESCRIPTION OF THE DISCLOSURE

It should be understood that both the general description above and the detailed description below are provided by way of example and illustration only, and are not intended to limit the scope of the claims of the present disclosure.

Unless otherwise defined, all technical and scientific terms used herein have the meanings commonly understood by those skilled in the art to which the present disclosure pertains.

The articles "a," "an," and "any" used in the present disclosure refer grammatically to one or more (e.g., at least one).

The primary objective of the present disclosure is to provide a hemostatic composition comprising an amino acid-metal complex, where the amino acid is glutamic acid or aspartic acid, and the metal is a divalent metal ion.

Specifically, the divalent metal ion is magnesium, manganese, cobalt, zinc, or calcium. In embodiments of the present disclosure, the preferred divalent metal ion is magnesium.

Specifically, the complex formed by the magnesium ion with glutamic acid or aspartic acid has the structures respectively shown in formulas (I) and (II) below:

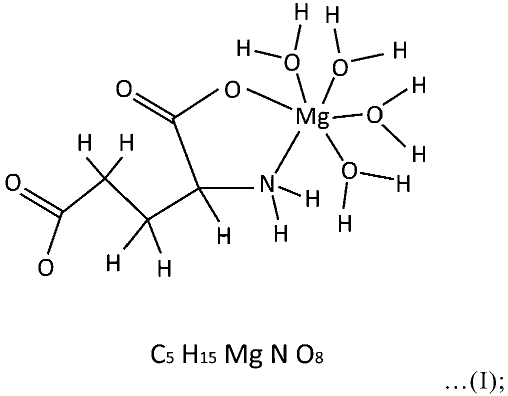

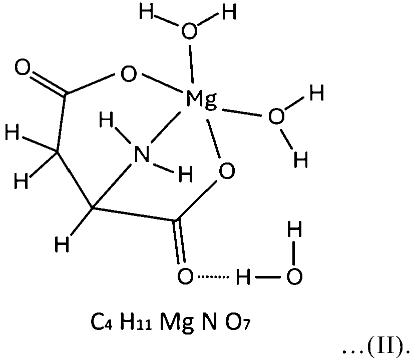

In the present disclosure, the term "complex" refers to a complex formed by two or more chemical substances through coordinate bonds (coordinate covalent bonds), Coulombic electrostatic interactions (e.g., ion-ion, ion-dipole, etc.), hydrogen bonds, van der Waals forces, hydrophobic interactions, or other intermolecular interactions, including but not limited to chelates.

In the present disclosure, the term "chelates" refers to a compound formed when a ligand binds to the same metal ion through two or more binding sites simultaneously. Specifically, in the present disclosure, the metal ion forms a complex by chelating with the amino acid glutamic acid or aspartic acid, which is more stable than the metal ion in its free state, reduces nonspecific chelation, and minimizes the oxidative stress that may be induced.

In the present disclosure, the term "amino acid" refers to an organic compound having both an amino group and a carboxyl group, which is the basic unit constituting proteins. In the present disclosure, the amino acid is a single amino acid molecule, rather than a peptide formed by two or more amino acids linked through peptide bonds.

In the present disclosure, the term "divalent metal ion" refers to an ion carrying two positive charges formed after a metal atom loses two electrons. Specifically, this type of metal ion can form a stable coordination structure with a ligand (such as a carboxyl group or an amino group) of an amino acid, thereby forming an amino acid-metal complex. Depending on the valence, metal ions possess different capabilities to chelate with amino acids; generally, a higher valence represents a greater positive charge density, meaning it is easier to attract the negatively charged ligands of amino acids, thereby resulting in a stronger chelating capability. However, trivalent metal ions, which have a higher valence, exhibit extremely high stability but may consequently reduce bioavailability, and in a solution (an electron-donating environment), they may oxidize the side chains of amino acids, causing a change in or destruction of the configuration of the chelate. Specifically, in the embodiments of the present disclosure, the selection of divalent metal ions helps achieve a balance between stability and biocompatibility, making the formed complex suitable for application in a physiological environment.

In the present disclosure, the hemostatic composition as described above does not contain chloride ions, sulfate ions, irritating salts, or antibiotics.

In the present disclosure, the term "irritating salts" refers to salts added to pharmaceuticals or preparations for antibacterial, pH adjustment, buffering, or anticoagulant purposes, but which cause irritation, corrosion, excessive osmotic pressure, or cytotoxicity in solution, where the irritating salts include, but are not limited to, silver nitrate, potassium nitrate, aluminum acetate, zinc acetate, sodium citrate, potassium citrate, sodium phosphate, potassium phosphate, or sodium carbonate.

In the present disclosure, the amino acid-metal complex in the hemostatic composition can be directly applied to wound treatment, and can also be combined with other pharmaceutically acceptable excipients, such as, but not limited to, water, alcohols, glycols, preserving agents, antioxidants, solvents, emulsifiers, suspending agents, decomposers, binding agents, excipients, stabilizing agents, chelating agents, diluents, gelling agents, preservatives, lubricants, absorption enhancers, active agents, humectants, odor absorbers, fragrances, pH adjusting agents, occlusive agents, emollients, thickeners, solubilizing agents, penetration enhancers, anti-irritants, colorants, propellants, or surfactants, to form a hemostatic composition in the form of a powder, tablet, dressing, spray, or gel.

In the present disclosure, the amino acid-metal complex may be a freeze-dried powder.

In the present disclosure, the pH value of the amino acid-metal complex after redissolving in water is 6.0, 6.5, 7.0, 7.5, 8.0, 8.5, 9.0, or any positive value, or any range formed by any two positive values, between 6.0 and 9.0; in a preferred embodiment, the pH value is 6.5 to 8.5.

In the present disclosure, the amino acid-metal complex is a freeze-dried magnesium aspartate powder, and exhibits an amorphous form, a partially crystalline form, or a mixture thereof in X-ray diffraction analysis.

In the present disclosure, the freeze-dried magnesium aspartate powder comprises a peak at a 2θ value of 6.9° ± 0.2° in an X-ray powder diffraction pattern.

In the present disclosure, the amino acid-metal complex comprises a magnesium aspartate crystalline form, which is formed by the magnesium aspartate complex under an alkaline condition.

The present disclosure also provides a use of the hemostatic composition described above for improving bleeding symptoms.

Specifically, the hemostatic composition of the present disclosure comprising the amino acid-metal complex has better stability and lower irritation compared to the prior art. Compared to commercially available hemostatic materials that may involve risks of allergic reactions, exothermic reactions during action causing wound burns, or significant decreases or even ineffectiveness of hemostatic effect under specific physiological conditions (such as individual shock, hypothermia, taking anticoagulant medications, or low coagulation factors due to severe blood loss), the composition of the present disclosure can maintain good hemostatic effect under different conditions.

The amino acid-metal complex used in the present disclosure does not cause allergic reactions, and because both amino acids and metal ions are substances that are easily absorbed by the human body, it has higher biocompatibility. Furthermore, it does not form foreign residue on the wound that requires additional removal after use, improving wound comfort and preventing potential rebleeding or infection during cleaning. In addition, the amino acid-metal complex of the present disclosure also shows significant improvement in its effect on clotting time and blood loss.

### Embodiments

In the present embodiment of the disclosure, the aforementioned glutamic acid, aspartic acid, and a highly safe metal source, such as oxides, hydroxides, or pure metals, are used to calculate the required molar number based on the molecular weights of glutamic acid (147.13 g/mol), aspartic acid (133.1 g/mol), magnesium ions (Mg²⁺) (24.31 g/mol), and calcium ions (Ca²⁺) (40.08 g/mol), and then chelated using methods well known to those skilled in the art.

Specifically, the sources of magnesium or calcium ions include, but are not limited to, metal compounds containing magnesium or calcium ions. Subsequently, the resulting amino acid-metal complex can be further dried to remove the solvent, where the drying process may include freeze-drying or other suitable methods. The resulting solid product can be further pulverized or ground to form a powder, and can be further prepared into powder, gel, spray, or other dosage forms suitable for hemostatic purposes.

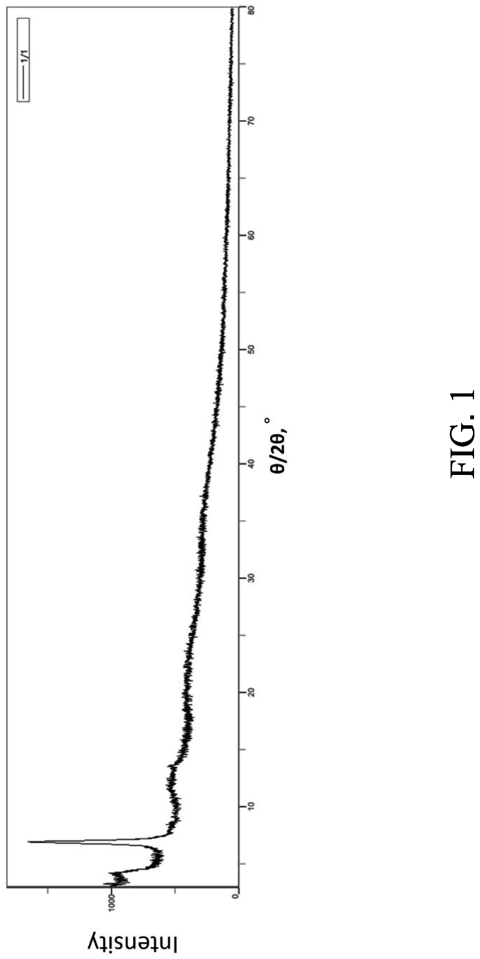

In one embodiment, aspartic acid and a magnesium source are reacted in a molar ratio of approximately 2:1 to form an aqueous solution of magnesium aspartate complex with a pH of approximately 7.5, which is then freeze-dried to form a powder. The resulting freeze-dried powder, after redissolving in water, still forms a nearly neutral magnesium aspartate complex solution. X-ray powder diffraction analysis of the freeze-dried powder revealed identifiable diffraction features in the low-angle region, including a peak at a 2θ value of 6.9° ± 0.2°, while exhibiting a broad diffraction background in the higher-angle region (FIG. 1). The results show that the freeze-dried powder of the magnesium aspartate complex of the present disclosure can exhibit physicochemical characteristics of an amorphous form, a partially crystalline form, a hydrated form, a polycrystalline form, or a mixture thereof.

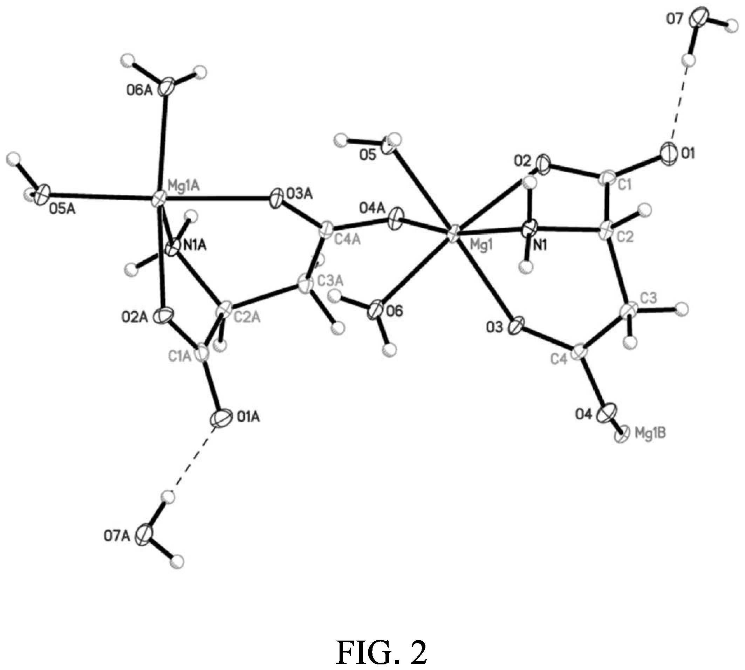

In another embodiment, adjusting the aforementioned magnesium aspartate complex solution to alkaline conditions, such as pH 10, can induce the formation of magnesium aspartate crystals. Specifically, other amino acid-metal complexes, such as magnesium glutamate crystals, can also be generated by adjusting the pH value. Single-crystal X-ray diffraction analysis confirmed that the crystals have a structure in which magnesium and aspartic acid are coordinated (FIG. 2). The crystals obtained under the above alkaline conditions can serve as one piece of structural evidence of the magnesium aspartate complex of the present disclosure; however, the hemostatic composition of the present disclosure is not limited to this crystal form, and may also include an amorphous form, a partially crystalline form, a hydrated form, a polycrystalline form, or a mixture thereof.

Other crystallographic data and refinement parameters are listed in Tables 1 and 2 below.

**Table 1. Crystallographic data and refinement parameters of magnesium**

| | |
|---|---|
| aspartate | |
| **Sample No.** | i20021 |
| **Empirical formula** | C₄H₁₁MgNO₇ |
| **Formula Weight** | 209.45 |
| **Temperature** | 100(2)K |
| **Wavelength** | 1.54178 Å |
| **Crystal system, Space group** | Tetragonal system ^{,} *P*2₁2₁2₁ |
| **Unit cell dimensions** | a = 7.7939(2) Å |
| | b = 9.3562(3) Å |
| | c = 11.5658(3) Å |
| | α = 90° |
| | β = 90° |
| | γ = 90° |
| **Unit cell Volume** | 843.39(4) Å³ |
| **Z, number of formula units in the unit cell, Calculated Density** | 4 ^{,} 1.650 mg/m³ |
| **Absorption Coefficient** | 2.029 mm⁻¹ |
| **F(000), number of electrons in the unit cell** | 440 |
| **Crystal Size** | 0.236 × 0.102 × 0.066 mm³ |
| **2 Theta Range of Data Collection** | 6.083 to 74.423° |
| **Limiting Indices** | -9 ≤ h ≤ 9 |
| | -11 ≤ k ≤ 11 |
| | -14 ≤ 1 ≤ 14 |
| **Reflections Collected/ Independent Reflections** | 11802/1718 [*R*ᵢₙₜ = 0.0445] |
| **Refinement Method** | Full-Matrix Least Squares on *F²* |
| **Completeness** | 99.6 % |
| **Data/Restraints/Parameters** | 1718/13/142 |
| **Goodness-of-fit on *F²*** | 1.141 |
| **Final *R* indices [I ≥ 2sigma(I)]** | *R*₁ =0.0409, *wR*₂ = 0.1096 |
| **Final *R* indices [All Data]** | *R*₁ = 0.0411, *wR*₂ = 0.1099 |
| **Largest diff. peak and hole** | 0.503, -0.589 e.Å⁻³ |

**Table 2. Crystallographic data and refinement parameters of magnesium glutamate**

| **Sample No.** | ic22844 |
|---|---|
| **Empirical formula** | C₅H₁₅MgNO₈ |
| **Formula Weight** | 241.49 |
| **Temperature** | 150(2)K |
| **Wavelength** | 1.54178 Å |
| **Crystal system, Space group** | Tetragonal system ^{,} *P*2₁2₁2₁ |
| **Unit cell dimensions** | a = 7.8180(3) Å |
| | b = 10.1107(3) Å |
| | c = 13.1435(4) Å |
| | α = 90° |
| | β = 90° |
| | γ = 90° |
| **Unit cell Volume** | 1038.93(6) Å³ |
| **Z, number of formula units in the unit cell, Calculated Density** | 4 ^{,} 1.544 mg/m³ |
| **Absorption Coefficient** | 1.800 mm⁻¹ |
| **F(000), number of electrons in the unit cell** | 512 |
| **Crystal Size** | 0.336 × 0.233 × 0.152 mm³ |
| **2 Theta Range of Data Collection** | 5.520 to 79.417° |
| **Limiting Indices** | -8 ≤ h ≤ 9 |
| | -12 ≤ k ≤ 11 |
| | -16 ≤ 1 ≤ 16 |
| **Reflections Collected/ Independent Reflections** | 17582/2219 [*R*ᵢₙₜ = 0.0390] |
| **Refinement Method** | Full-Matrix Least Squares on *F²* |
| **Completeness** | 99.7 % |
| **Data/Restraints/Parameters** | 2219/0/160 |
| **Goodness-of-fit on *F²*** | 1.129 |
| **Final *R* indices [I ≥ 2sigma(I)]** | *R*₁ =0.0267, *w*R₂ = 0.0716 |
| **Final *R* indices [All Data]** | *R*₁ = 0.0268, *w*R₂ = 0.0717 |
| **Largest diff. peak and hole** | 0.321, -0.262 e.Å⁻³ |

Example 1: Hemostatic effect of amino acid-metal complex in animal experiments

In the present embodiment, 27 male ICR mice aged 6-8 weeks and weighing 20-25 g were used. The mice were randomly divided into 9 groups of 3 mice each including a blank control group using gauze, a reference control group using tranexamic acid, and experimental groups treated with calcium glutamate, calcium aspartate, zinc aspartate, magnesium glutamate, magnesium aspartate, cobalt glutamate, and cobalt aspartate.

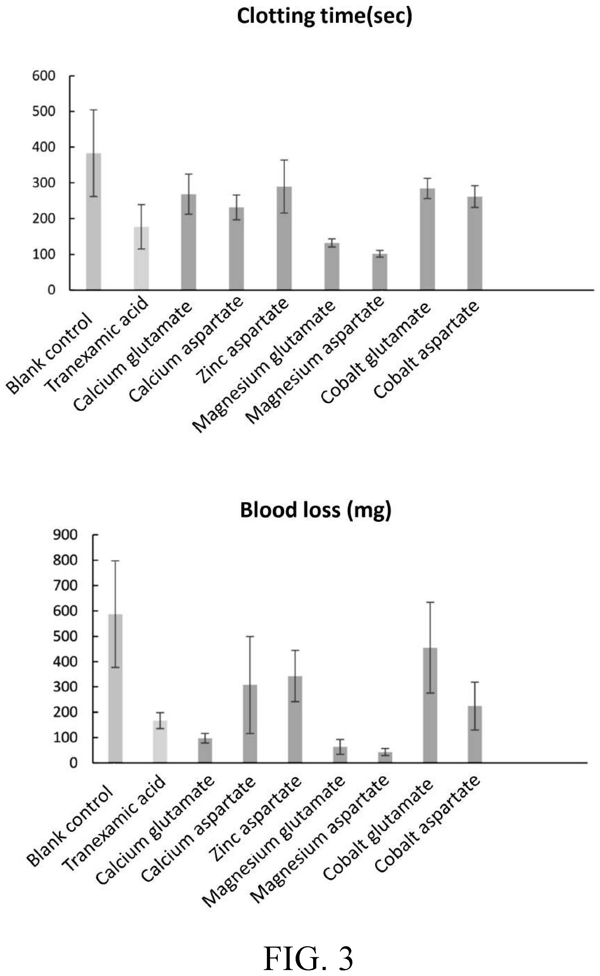

The tails of mice were amputated, and the wounds were completely covered with the amino acid-metal complex powder of the above groups using minimal pressure. Clotting time and blood loss were recorded for each group. All procedures were performed under aseptic conditions. Specifically, in the present disclosure, "minimal pressure" refers to uniformly covering the wound with the complex powder without applying pressure to the wound, thus avoiding affecting the hemostasis result. The results are shown in FIG. 3, and the quantitative results are shown in Table 3 below.

**Table 3. Hemostatic effects of different test materials on mouse wounds**

| | Test material | Clotting time (s) | Blood loss (mg) |
|---|---|---|---|
| Control group | Blank control group | 383.0 ± 121.2 | 587.6 ± 210.0 |
| | Tranexamic acid | 177.0 ± 62.0 | 166.6 ± 31.2 |
| Experimental group | Calcium glutamate | 268.3 ± 56.3 | 97.3 ± 19.1 |
| | Calcium aspartate | 231.3 ± 34.6 | 307.7 ± 191.9 |
| | Zinc aspartate | 289.7 ± 74.1 | 343.1 ± 101.6 |
| | Magnesium glutamate | 132.0 ± 11.3 | 63.2 ± 29.4 |
| | Magnesium aspartate | 101.7 ± 9.5 | 42.9 ± 13.8 |
| | Cobalt glutamate | 284.3 ± 28.3 | 454.9 ± 179.9 |
| | Cobalt aspartate | 261.7 ± 30.4 | 224.5 ± 94.8 |

In the present embodiment, 33 male SD rats aged 8-9 weeks and weighing 210-280 g were used. The rats were randomly divided into 11 groups of 3 rats each including a blank control group using gauze, a reference control group using commercially available CeloX, QuickClot, and tranexamic acid, and experimental groups treated with calcium glutamate, calcium aspartate, zinc aspartate, magnesium glutamate, magnesium aspartate, cobalt glutamate, and cobalt aspartate.

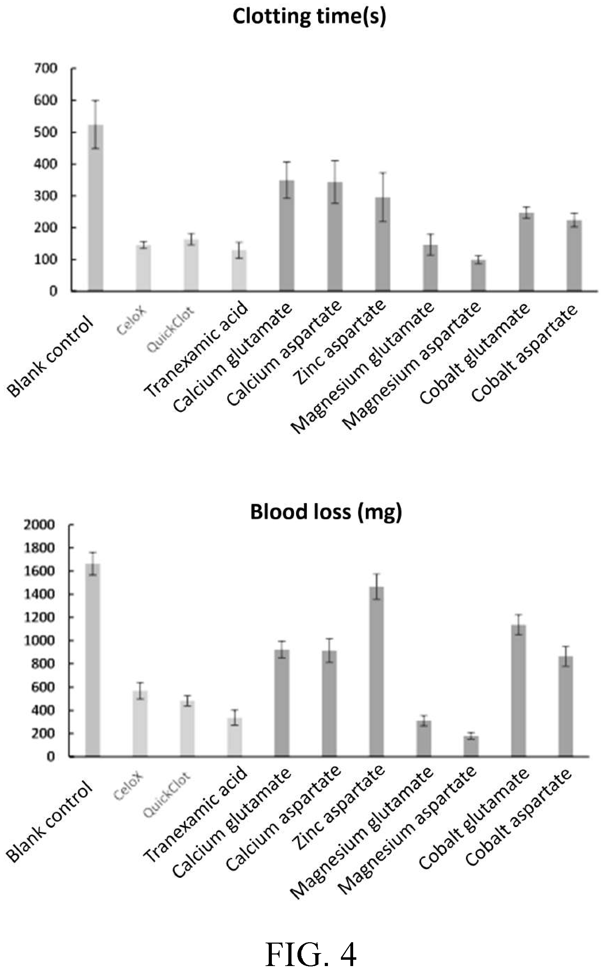

The tails of rats were amputated, and the wounds were completely covered with the test materials of the above groups using minimal pressure. Clotting time and blood loss were recorded for each group. All procedures were performed under aseptic conditions. The results are shown in FIG. 4, and the quantitative results are shown in Table 4 below.

**Table 4. Hemostatic effects of different test materials on rat wounds**

| | Test material | Clotting time (s) | Blood loss (mg) |
|---|---|---|---|
| Control group | Blank control group | 524.3 ± 75.3 | 1664.7 ± 98.4 |
| | CeloX | 145.0 ± 10.3 | 570.3 ± 71.3 |
| | QuickClot | 164.0 ± 18.0 | 483.3 ± 46.0 |
| | Tranexamic acid | 129.3 ± 24.9 | 336.7 ± 65.3 |
| Experimental group | Calcium glutamate | 349.5 ± 56.9 | 890.0 ± 64.7 |
| | Calcium aspartate | 343.8 ± 67.4 | 748.0 ± 68.0 |
| | Zinc aspartate | 296.0 ± 76.6 | 1466.0 ± 110.2 |
| | Magnesium glutamate | 146.3 ± 32.8 | 311.0 ± 44.7 |
| | Magnesium aspartate | 99.5 ± 13.2 | 179.7 ± 29.2 |
| | Cobalt glutamate | 247.3 ± 17.2 | 1137.0 ± 85.7 |
| | Cobalt aspartate | 224.3 ± 21.6 | 864.7 ± 87.0 |

As shown in FIGS. 3 and 4 and Tables 3 and 4, compared with the blank control group, the clotting time and blood loss of tranexamic acid and the experimental groups were both reduced; among them, magnesium glutamate and magnesium aspartate achieved the same clotting time and blood loss as tranexamic acid; in other words, the amino acid-metal complex of the present disclosure can still produce good hemostasis while avoiding possible side effects such as thrombosis caused by tranexamic acid.

Compared with commercially available CeloX, QuickClot or tranexamic acid, magnesium glutamate and magnesium aspartate of the present disclosure have shorter clotting time and less blood loss; in other words, the amino acid-metal complex of the present disclosure can avoid the risks of allergies, infections or individual limitations caused by components such as chitosan or kaolin, while also having better hemostatic effects.

In summary, the present disclosure provides a hemostatic composition containing an amino acid-metal complex, which can be used to improve bleeding symptoms. Compared to existing hemostatic materials, the hemostatic composition of the present disclosure has a simple composition, mainly consisting of amino acids and metal ions. It does not rely on pharmacological mechanisms of action, making it suitable for bleeding control. Furthermore, it is less likely to cause allergic reactions or burning sensations, exhibiting better biocompatibility and reducing the risk of allergic reactions and tissue irritation. In addition, the hemostatic composition of the present disclosure also significantly improves clotting time and blood loss control.

The present disclosure has been disclosed above in a preferred embodiment; however, those skilled in the art will understand that this embodiment is intended merely to illustrate the present disclosure and should not be construed as limiting the scope of the disclosure. It should be noted that any variations and substitutions equivalent to this embodiment should be considered to fall within the scope of the disclosure. Accordingly, the scope of protection of the present disclosure shall be defined by the claims.

## Claims

1. A hemostatic composition comprising an amino acid-metal complex, wherein the amino acid is glutamic acid or aspartic acid, and the metal is a divalent metal ion.

2. The hemostatic composition according to claim 1, wherein the divalent metal ion is magnesium, manganese, cobalt, zinc, or calcium.

3. The hemostatic composition according to claim 1, wherein the amino acid-metal complex is magnesium glutamate or magnesium aspartate, structures of which are respectively represented by the following formula (I) and formula (II) below:

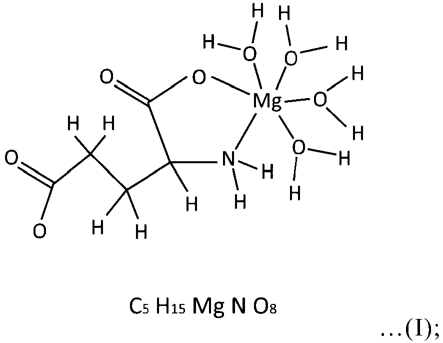

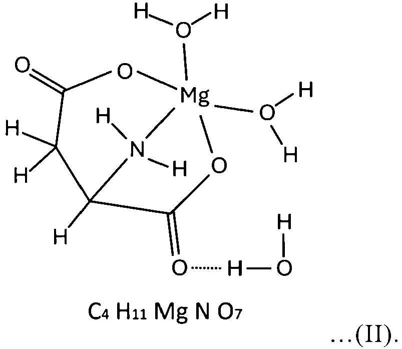

4. The hemostatic composition according to claim 1, wherein the hemostatic composition does not contain chloride ions, sulfate ions, irritating salts, or antibiotics.

5. The hemostatic composition according to claim 1, wherein the hemostatic composition is a powder, tablet, dressing, spray, or gel.

6. The hemostatic composition according to claim 1, wherein the amino acid-metal complex is a freeze-dried powder.

7. The hemostatic composition according to claim 1, wherein a pH value of the amino acid-metal complex after redissolving in water is 6.5 to 8.5.

8. The hemostatic composition according to claim 1, wherein the amino acid-metal complex is a freeze-dried magnesium aspartate powder, and exhibits an amorphous form, a partially crystalline form, or a mixture thereof in X-ray diffraction analysis.

9. The hemostatic composition according to claim 1, wherein the amino acid-metal complex is a freeze-dried magnesium aspartate powder, and comprises a peak at a 2θ value of 6.9° ± 0.2° in an X-ray powder diffraction pattern.

10. The hemostatic composition according to claim 1, wherein the amino acid-metal complex comprises a crystalline form of magnesium aspartate, the crystalline form being formed by a magnesium aspartate complex under an alkaline condition.

11. Use of the hemostatic composition according to claim 1, wherein the use is for improving bleeding symptoms.
